(19) Europäisches Patentamt
European Patent Office
Office européen des brevets

(11) Veröffentlichungsnummer : **0 021 356**
**B1**

(12) # EUROPÄISCHE PATENTSCHRIFT

(45) Veröffentlichungstag der Patentschrift : **16.02.83**

(21) Anmeldenummer : **80103439.8**

(22) Anmeldetag : **20.06.80**

(51) Int. Cl.³ : **C 07 C 49/553**, C 07 C 45/69, C 11 B 9/00, C 07 C 29/40, C 07 C 35/06, C 07 C 1/24, C 07 C 13/12

(54) **4(5)-Acetyl-7,7,9(7,9,9)-trimethyl-bicyclo(4.3.0)-non-1-en, dessen Herstellung und Verwendung als Riechstoff, sowie dieses enthaltende Riechstoffkompositionen.**

(30) Priorität : **25.06.79 DE 2925622**

(43) Veröffentlichungstag der Anmeldung : **07.01.81 Patentblatt 81/01**

(45) Bekanntmachung des Hinweises auf die Patenterteilung : **16.02.83 Patentblatt 83/07**

(84) Benannte Vertragsstaaten : **AT BE CH DE FR GB IT LI NL SE**

(56) Entgegenhaltungen :
**FR A 2 316 922**
**CHEMICAL ABSTRACTS, Band 88, Nr. 11, 13. März 1978, Zusammenfassung Nr. 74232r. Seite 473, Columbus, Ohio, US**

(73) Patentinhaber : **Henkel Kommanditgesellschaft auf Aktien**
**Postfach 1100 Henkelstrasse 67**
**D-4000 Düsseldorf 1 (DE)**

(72) Erfinder : **Bruns, Klaus, Dr.**
**Notburgaweg 6**
**D-4150 Krefeld-Traar (DE)**
Erfinder : **Weber, Ursula**
**Am Roerfeld 2**
**D-4175 Wachtendonk (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

« 4(5)-Acetyl-7,7,9(7,9,9)-trimethyl-bicyclo [4.3.0] non-1-en, dessen Herstellung und Verwendung als Riechstoff, sowie dieses enthaltende Riechstoffkompositionen »

Es wurde gefunden, daß das Isomerengemisch 4(5)-Acetyl-7,7-9(7,9,9)-trimethyl-bicyclo [4.3.0] non-1-en einen wertvollen neuen Riechstoff von kräftigem, warmem Ambra-Duft mit holziger Tabakblatt-, Methyljonon- und Thuja-Note sowie außergewöhnlicher Haftfestigkeit darstellt.

Die Herstellung des erfindungsgemäßen neuen Isomerengemisches erfolgt nach an sich bekannten Syntheseverfahren der organischen Chemie. Als Ausgangsmaterial für die Synthese dient das Isomerengemisch 2,2,4(2,4,4)-Trimethylcyclopentanon (I), das mittels der Reaktion nach Grignard zum 1-Vinyl-1-hydroxy-2,2,4(2,4,4)-trimethylcyclopentan (II) umgesetzt wird. Das als Ausgangsmaterial dienende Trimethylcyclopentanon liegt stets als Isomerengemisch vor und ist in Gestalt einer strukturell einheitlichen Verbindung nich erhältlich. Durch Dehydratisierung des 1-Vinyl-1-hydroxy-2,2,4(2,4,4)trimethylcyclopentans mittels p-Toluolsulfonsäure gelangt man zum 1-Vinyl-2,2,4(2,4,4)-trimethylcyclopent-1-en (III). Dieses wird durch Umsetzung mit Methylvinylketon nach Diels-Alder in die gewünschte Verbindung 4(5)-Acetyl-7,7,9-(7,9,9)-trimethyl-bicyclo [4.3.0] non-1-en (IV) überführt.

Es handelt sich dabei um ein Stellungs-/Stereoisomerengemisch bezogen auf die Kohlenstoffatome 4, 5, 6, 7, 8 und 9. Die Herstellung verläuft gemäß nachstehendem Reaktionsschema :

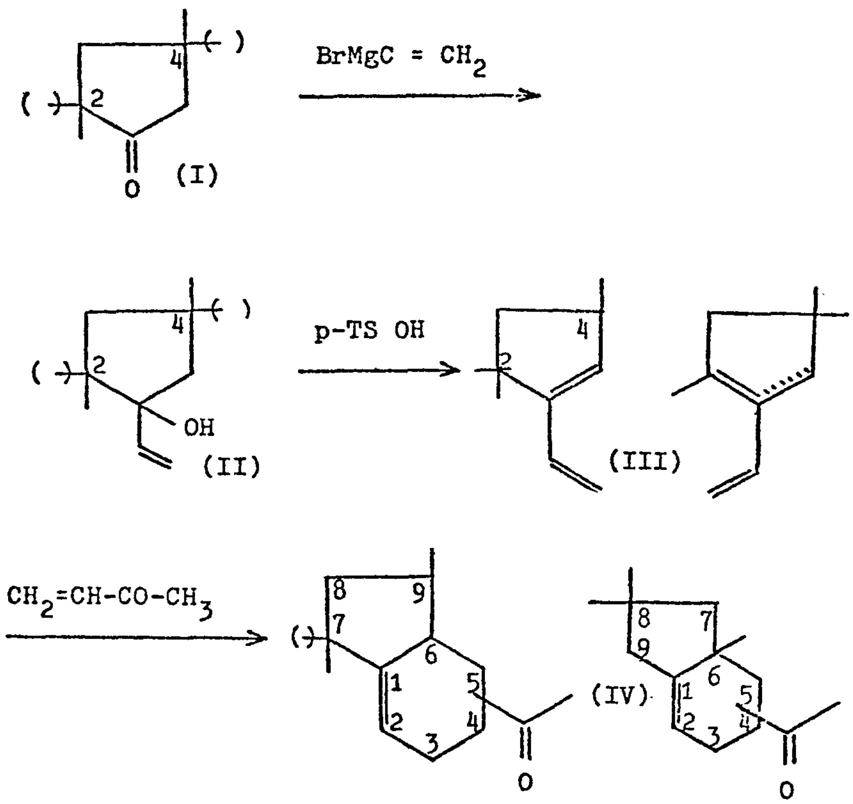

(I) (II) (III) (IV)

Der neue Riechstoff stellt demnach ein Gemisch folgender Isomerer dar :

4-Acetyl-7,7,9-trimethyl-bicyclo [4.3.0] non-1-en,
5-Acetyl-7,7,9-trimethyl-bicyclo [4.3.0] non-1-en,
4-Acetyl-7,9,9-trimethyl-bicyclo [4.3.0] non-1-en,
5-Acetyl-7,9,9-trimethyl-bicyclo [4.3.0] non-1-en,

wobei Acetylgruppe und Ringverknüpfung $C_6/C_7$ axiale und äquatoriale Konfiguration einnehmen können.

Das Isomerengemisch 4(5)-Acetyl-7,7,9(7,9,9)-trimethyl-bicyclo [4.3.0] non-1-en zeichnet sich durch einen kräftigen, warmen Ambra-Duft mit holziger Tabakblatt-, Methyljonon-, und Thuja-Note, sowie eine außergewöhnliche Haftfestigkeit aus. Ein weiterer Vorteil ist seine sehr gute Kombinationsfähigkeit zu neuartigen Kompositionen, denen es gleichfalls eine besondere Haftfestigkeit verleiht.

Das Isomerengemisch 4(5)-Acetyl-7,7,9(7,9,9)-trimethyl-bicyclo [4.3.0] non-1-en kann mit anderen Riechstoffen in den verschiedensten Mengenverhältnissen zu neuen Riechstoffkompositionen gemischt werden. Im allgemeinen wird sich sein Anteil in den Riechstoffkompositionen in den Mengen von 1 bis 50 Gewichtsprozent, bezogen auf die gesamte Komposition, bewegen. Derartige Kompositionen können zur Parfümierung von kosmetischen Präparaten wie Cremes, Lotionen, Duftwässern, Aerosolen, Toiletteseifen als auch in der Extrait-Parfümerie verwendet werden.

Sie können aber auch zur Geruchsverbesserung technischer Produkte wie Wasch- und Reinigungsmitteln, Weichspülern, Textilbehandlungsmitteln eingesetzt werden. Zur Parfümierung der verschiedenen Produkte werden diesen die Kompositionen im allgemeinen in Konzentrationen von 0,05 bis 2 Gewichtsprozent, bezogen auf das gesamte Produkt, zugesetzt.

Die nachfolgenden Beispiele sollen den Gegenstand der Erfindung näher erläutern, ohne ihn jedoch hierauf zu beschränken.

## Beispiele

Herstellung von 4(5)-Acetyl-7,7,9(7,9,9)-trimethyl-bicyclo [4.3.0] non-1-en

a) Herstellung von 1-Vinyl-1-hydroxy-2,2,4(2,4,4)-trimethylcyclopentan.

Aus 1,4 Mol Magnesium und 1,4 Mol Vinylbromid wurde in 450 ml trockenem Tetrahydrofuran in üblicher Weise die entsprechende Grignard-Lösung hergestellt. Unter Rühren wurde eine Lösung von 1,3 Mol 2,2,4(2,4,4)-Trimethylcyclopentanon (GC ca. 65/35 %) in 160 ml Tetrahydrofuran zugetropft. Anschließend wurde das Gemisch 12 Stunden am Rückflüß erhitzt. Nach dem Abkühlen wurde mit 10 %iger Schwefelsäure zersetzt und wie üblich aufgearbeitet. Nach Abdestillieren des Lösungsmittels wurde das Reaktionsprodukt im Wasserstrahlvakuum destilliert. Das erhaltene Reaktionsprodukt stellte eine farblose Flüssigkeit mit folgenden Kennzahlen dar :

$$Kp_{0,02\ bar}\ 65\text{-}71\ °C$$

IR(Film) : 3 460/cm (OH) ; 3 085, 1 640, 1 412, 995, 919/cm

($—CH = CH_2$)

b) Herstellung von 1-Vinyl-2,2,4(2,4,4)-trimehtylcyclopent-1-en.

0,1 Mol 1-Vinyl-1-hydroxy-2,2,4(2,4,4)-trimethylcyclopentan wurden in 30 ml Chloroform in Gegenwart von 0,002 Mol p-Toluolsulfonsäure 15 Minuten unter Rückfluß am Wasserabscheider erhitzt. Danach wurden 1 g Natriumcarbonat zugesetzt und das Lösungsmittel abdestilliert. Der Rückstand wurde in Ether aufgenommen, mit 2n-Sodalösung extrahiert und mit Wasser neutral gewaschen. Nach Abdestillieren des Lösungsmittels wurde im Wasserstrahlvakuum destilliert. Das erhaltene Reaktionsprodukt stellt eine farblose Flüssigkeit mit folgenden Kennzahlen dar :

$$K_{p0,12\ bar}\ 78\text{-}84\ °C$$

IR (Film) : 3 080, 1 635, 1 415, 985, 893/cm ($—CH = CH_2$) ;
1 588/cm (Konjugation), 852, 840/cm

($>C = CH—$)

c) Herstellung von 4(5)-Acetyl-7,7,9(7,9,9)-trimethyl-bicyclo [4.3.0] non-1-en.

1,1 Mol 1-Vinyl-2,2,4(2,4,4)-trimethylcyclo-pent-1-en und 1,2 Mol Methylvinylketon wurden unter Rühren 3 Stunden lang am Rückfluß erhitzt. Das erhaltene Rohprodukt wurde im Ölpumpenvakuum fraktioniert destilliert. Das hierbei gewonnene Isomerengemisch von 4(5)-Acetyl-7,7,9(7,9,9)-trimethylbicyclo [4.3.0]-non-1-en hatte folgende Kennzahlen :

$$K_{p0,002}\ bar\ 94\text{-}103\ °C$$

IR (Film) : 1 710/cm (Keton) ; 1 155/cm ($—COCH_3$)
$^1$H-NMR($CDCl_3$) : 0,76-1,08 ppm (m, 9H) ($C—CH_3$) ;
2,16 ppm (m, 3H) ($CO—CH_3$)
5,34 ppm (1H, m) ($C = CH$).

Das Produkt besitzt einen kräftigen, warmen Ambra-Duft mit holziger Tabakblatt-, Methyljonon- und Thuja-Note.

## Beispiel 1

Riechstoffkomposition Tabak-Base

| | |
|---|---|
| 4(5)-Acetyl-7,7,9(7,9,9)-trimethyl-bicyclo [4.3.0] non-1-en | 300 Gew.-Teile |
| Geraniol | 120 Gew.-Teile |
| Vetiverol | 100 Gew.-Teile |
| Cedrylacetat | 50 Gew.-Teile |
| Cedernholzöl | 50 Gew.-Teile |
| Santalol | 50 Gew.-Teile |
| Eichenmoos Resin | 40 Gew.-Teile |
| Guajylacetat | 30 Gew.-Teile |
| Gamma-Methyljonon | 30 Gew.-Teile |
| Benzophenon | 30 Gew.-Teile |
| Galaxolid | 30 Gew.-Teile |
| Patchouliöl | 30 Gew.-Teile |
| Olibanum Resin | 25 Gew.-Teile |
| Isobutylsalicylat | 25 Gew.-Teile |
| Bergamotteöl | 20 Gew.-Teile |
| Lavendelöl | 20 Gew.-Teile |
| Methylnaphthylketon | 20 Gew.-Teile |
| Cumarin | 20 Gew.-Teile |
| Cinnamylacetat | 10 Gew.-Teile |
| | 1.000 Gew-Teile |

Beispiel 2

Chypre-Komposition

| | |
|---|---|
| 4(5)-Acetyl-7,7,9(7,9,9)-trimethyl-bicyclo [4.3.0] non-1-en | 200 Gew.-Teile |
| Orangenöl, Florida | 280 Gew.-Teile |
| Bergamotteöl | 200 Gew.-Teile |
| Jasmin Absolue | 50 Gew.-Teile |
| Vetiverylacetat | 40 Gew.-Teile |
| Cumarin | 30 Gew.-Teile |
| Eichenmoos Absolue | 30 Gew.-Teile |
| Sandelholzöl ostind. | 30 Gew.-Teile |
| Ketonmoschus | 30 Gew.-Teile |
| Patchouliöl | 30 Gew.-Teile |
| Labdanum Resin | 20 Gew.-Teile |
| Vetiveröl | 20 Gew.-Teile |
| Ambroxan, Henkel | 20 Gew.-Teile |
| Basilikumöl | 5 Gew.-Teile |
| Estragonöl | 5 Gew.-Teile |
| Vanillin | 5 Gew.-Teile |
| Zibet Absolue | 2 Gew.-Teile |
| Methylnonylacetaldehyd 10 % | 2 Gew.-Teile |
| Undecylenaldehyd | 1 Gew.-Teil |
| | 1.000 Gew.-Teile |

**Ansprüche**

1. Isomerengemisch 4(5)-Acetyl-7,7,9(7,9,9)-trimethyl-bicyclo [4.3.0]-non-1-en.

2. Verfahren zur Herstellung des Isomerengemisches 4(5)-Acetyl-7,7,9(7,9,9)-trimethyl-bicyclo [4.3.0]-non-1-en durch Umsetzen in erster Stufe von 2,2,4(2,4,4)-Trimethylcyclopentanon (I) nach Grignard mit Vinylmagnesiumbromid zum 1-Vinyl-1-hydroxy-2,2,4(2,4,4)-trimethylcyclopentan (II), dessen Dehydratisierung mittels p-Toluolsulfonsäure in zweiter Stufe zum 1-Vinyl-2,2,4(2,4,4)-trimethylcyclopent-1-en (III) und weitere Umsetzung des (III) in dritter Stufe mit Methylvinylketon nach Diels-Alder zum 4(5)-Acetyl-7,7,9(7,9,9)-trimethyl-bicyclo [4.3.0]-non-1-en in Form des Stellungs-/Stereoisomerengemisches.

3. Verwendung des Isomerengemisches 4(5)-Acetyl-7,7,9(7,9,9)-trimethyl-bicyclo [4.3.0]-non-1-en als Riechstoff.

4. Riechstoffkompositionen, dadurch gekennzeichnet, daß sie das Isomerengemisch 4(5)-Acetyl-7,7,9(7,9,9)-trimethyl-bicyclo [4.3.0]-non-1-en in einer Menge von 1-50 Gewichtsprozent, bezogen auf die gesamte Komposition, enthalten.

**Claims**

1. The isomer mixture 4(5)-acetyl-7,7,9(7,9,9)-trimethyl-bicyclo [4.3.0] non-1-ene.

2. A process for producing the isomer mixture 4(5)-acetyl-7,7,9(7,9,9)-trimethyl-bicyclo [4.3.0] non-1-ene by the Grignard reaction in a first stage of 2,2,4(2,4,4)-trimethyl cyclopentanone (1) with vinyl magnesium bromide to form 1-vinyl-1-hydroxy-2,2,4(2,4,4)-trimethyl cyclopentane (II), dehydrating (II) with p-toluene sulfonic acid in a second stage to form 1-vinyl-2,2,4(2,4,4)-trimethylcyclopent-1-ene (III) and subjecting (III) to a Diels-Alder reaction with methyl vinyl ketone in a third stage to form 4(5)-acetyl-7,7,9(7,9,9)-trimethyl-bicyclo [4.3.0.] non-1-ene in the form of the position/stereo-isomer mixture.

3. The use of the isomer mixture 4(5)-acetyl-7,7,9(7,9,9)-trimethyl-bicyclo [4.3.0] non-1-ene as a perfume.

4. Perfume compositions, characterised in that they contain the isomer mixture 4(5)-acetyl-7,7,9(7,9,9)-trimethyl-bicyclo [4.3.0] non-1-ene in a quantity of from 1 to 50 % by weight, based on the composition as a whole.

**Revendications**

1. Le mélange d'isomères 4(5)-acétyl-7,7,9(7,9,9)-triméthyl-bicyclo [4.3.0] non-1-ène.

2. Procédé de préparation du mélange d'isomères 4(5)acétyl-7,7,9(7,9,9)-triméthyl-bicyclo [4.3.0] non-1-ène par réaction dans un premier stade de la 2,2,4(2,4,4)-triméthylcyclopentanone (I) selon Grignard avec le bromure de vinylmagnésium, conduisant au 1-vinyl-1-hydroxy-2,2,4(2,4,4)-triméthylcyclopentane (II), déshydratation de ce dernier à l'aide de l'acide p-toluènesulfonique dans un deuxième stade conduisant au 1-vinyl-2,2,4(2,4,4)-triméthylcyclopent-1-ène (III) et réaction de ce dernier dans un troisième stade avec la méthylvinylcétone selon Diels-Alder, conduisant au 4(5)acétyl-7,7,9(7,9,9)-triméthyl-bicyclo [4.3.0] non-1-ène à l'état de mélange d'isomères de positions/stéréoisomères.

3. Utilisation du mélange d'isomères 4(5)-acétyl-7,7,9(7,9,9)-triméthyl-bicyclo [4.3.0] non-1-ène en tant que matière aromatique.

4. Compositions de parfums, caractérisées en ce qu'elles contiennent le mélange d'isomères 4(5)acétyl-7,7,9(7,9,9)-triméthyl-bicyclo [4.3.0] non-1-ène en quantité de 1 à 50 % du poids de la composition totale.